(19) Europäisches Patentamt
European Patent Office
Office européen des brevets

(11) EP 3 656 760 A1

(12) # EUROPÄISCHE PATENTANMELDUNG

(43) Veröffentlichungstag:
**27.05.2020 Patentblatt 2020/22**

(51) Int Cl.:
***C07C 319/12*** $^{(2006.01)}$ ***C07C 323/12*** $^{(2006.01)}$

(21) Anmeldenummer: **18207569.7**

(22) Anmeldetag: **21.11.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH**
**45128 Essen (DE)**

(72) Erfinder:
- **HIEROLD, Judith**
  **30161 Hannover (DE)**
- **KOPP, Andrea**
  **63839 Kleinwallstadt (DE)**
- **RAUTENBERG, Stephan**
  **53332 Bornheim (DE)**
- **RENNER, Christian**
  **63584 Gründau (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **LAGERSTABILE FORM VON 3-METHYLTHIOPROPIONALDEHYD**

(57) Die Erfindung betrifft eine Chemische Verbindung der Formel (I),

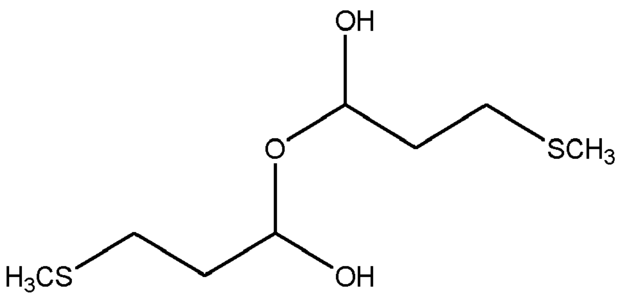

Zusammensetzungen umfassend 3- Methylthiopropionaldehyd, 3-Methylthio - 1,1-propandiol, eine Verbindung gemäß Formell und Wasser, desweiteren Verfahren zu deren Herstellung sowie deren Verwendung zur Herstellung von 2-Hydroxy-4-(methylthio)buttersäurenitril, Methioninhydantoin, Methionin und deren Verwendung als geschützte Form zur Lagerung und/oder zum Transport von 3-Methylthiopropionaldehyd.

EP 3 656 760 A1

## Beschreibung

Die vorliegende Erfindung betrifft eine Verbindung gemäß der Formel (I) mit dem Namen 1-(1'-Hydroxy-3'-(methylthio)propoxy)-3-(methylthio)-propan-1-ol, eine diese Verbindung umfassende Zusammensetzung und ein Verfahren zur Herstellung der Verbindung gemäß der allgemeinen Formel (I) aus 3-Methylthiopropionaldehyd (= 3-Methylmercaptopropionaldehyd, MMP) und Wasser. Ferner betrifft die vorliegende Erfindung auch ein Verfahren zur Herstellung von Methionin aus einer Verbindung der allgemeinen Formel (I) oder aus einer diese Verbindung umfassenden Zusammensetzung und die Verwendung einer Verbindung der allgemeinen Formel (I) zur Lagerung und/oder zum Transport von 3-Methylthiopropionaldehyd.

## Stand der Technik

3-Methylthiopropionaldehyd ist ein wichtiges Intermediat in der Herstellung von D,L-Methionin und seinem Hydroxy-Analogen 2-Hydroxy-4-methylthiobuttersäure, auch bekannt unter der Abkürzung MHA für Methionin-Hydroxy-Analog. Üblicherweise wird 3-Methylthiopropionaldehyd durch Umsetzung von Methylmercaptan mit Acrolein in einer Michael-Addition hergestellt. Die Patente GB 1618884 A, GB 1173174 A und GB 1166961 A offenbaren Verfahren zur direkten Herstellung von 3-Methylthiopropionaldehyd durch Umsetzung von Methylmercaptan mit Acrolein oder durch indirekten Herstellung von 3-Methylthiopropionaldehyd, bei der zunächst 3-Methylthiopropionaldehyd mit Methylmercaptan umgesetzt wird und das derart erhaltene Reaktionsprodukt direkt mit Acrolein zu 3-Methylthiopropionaldehyd umgesetzt wird. Die zitierten Dokumente thematisieren nicht, dass das erhaltene 3-Methylthiopropionaldehyd von geringer Lagerstabilität ist, so dass für diese Problematik dort keine Lösung bereitgestellt wird.

Das Problem der geringen Lagerstabilität von MMP durch Bildung höhermolekularer Schwersieder (summarisch als "Rückstand" bezeichnet, da diese bei einer einfachen Destillation als Destillationsrückstand zurückbleiben) ist mittlerweile bekannt. Die Rückstandsbildung ist dabei hauptsächlich auf die hohe Reaktivität der $\alpha$-aziden Aldehydfunktion zurückzuführen, welche z.B. intermolekulare Aldol-Additions- und Aldol-Kondensationsreaktionen begünstigt. Obwohl in der Produktion meist destilliertes MMP eingesetzt wird, um den Eintrag von z.T. auch farbintensiven Nebenprodukten zu vermeiden, erfolgen Zwischenlagerung und standortübergreifende Transporte meist notwendigerweise auf der deutlich stabileren Stufe des undestillierten Methylmercaptopropionaldehyds. Dieser muss wiederum mit meist pH-regulierenden Additiven versetzt werden, welche die Bildung von Rückstand weitgehend unterbinden.
Um das zur Synthese von Methionin genutzte Intermediat MMP lagerstabil und damit transportfähig zu machen, werden bisher unterschiedlichste Additive zugesetzt. Als Additiv verwendet beispielsweise WO 9313059 eine Mischung aus Triethanolamin und Ascorbinsäure, EP899258 A1 gesättigten Metallkomplexe bildende organische Säuren wie L-Weinsäure, JP 49116017 A N,N-Dialkylaniline, JP 10152467 A Eisenoxide oder EP 2813489 A1 nacheinander Amine und organische Säuren.

Vor dem weiteren Einsatz im Prozess werden diese Additive durch Destillation weitgehend abgetrennt. Destilliertes MMP (Rein-MMP) hat somit eine deutlich verringerte Lagerstabilität von wenigen Tagen und wird daher möglichst unverzüglich in der Produktion weiterverarbeitet und im Regelfall nicht mehr transportiert, um Verluste durch Nebenproduktbildung zu vermeiden.

An der Verwendung von Additiven ist von Nachteil, dass diese zunächst zugesetzt und im Anschluss wieder entfernt werden müssen. Benötigte man diese Zusätze nicht, liesen sich daher merklicher Aufwand und Kosten einsparen. Zusätzlich ist man durch die Limitierung, ausschließlich undestilliertes MMP transportieren zu können, weniger flexibel im logistischen Austausch von Intermediaten.

Die jüngere Patentanmeldung EP 3205643 A1 umgeht die Problematik der Additivverwendung durch die Verwendung von 1,3-Bis(methylthio)propan-1-ol, einem Additionsprodukt aus einem Molekül MMP und einem Molekül Methylmercaptan als stabile Lagerform für Methylmercaptopropionaldehyd und/oder Methylmercaptan.

Desweiteren löst EP 3339290 A1 die Problematik der Additivverwendung durch die Verwendung einer Zusammensetzung zur Lagerung und/oder zum Transport von 3-Methylmercaptopropionaldehyd und/oder Methylmercaptan, bei dem MMP mit Methylmercaptan umgesetzt wird zu der lagerstabilen Verbindung 1-(1,3-Bis(methylthio)propoxy)-3-(methylthio)propan-1-ol, einem Additionsprodukt aus 2 Molekülen MMP und einem Molekül Methylmercaptan sowie eine dieses Produkt enthaltende Zusammensetzung.

Nachteil dieser Lagerform wie auch der Lagerform 1,3-Bis(methylthio)propan-1-ol aus EP 3205643 A1 ist jedoch, dass diese Produkte nicht direkt weiter umgesetzt werden können zum Methionin, sondern vorher der darin gebundene Anteil an Methylmercaptan erst noch durch eine weitere Umsetzung mit Acrolein vollständig zu 3- Methylmercaptopropionaldehyd umgesetzt werden muss, was zusätzlichen Aufwand bedeutet. Gleichzeitig sind jedoch wegen des höheren Risikopotenzials gegenüber MMP selber die Sicherheitsanforderungen höher als bei Lagerung von MMP.

**Aufgabe**

Es bestand daher Bedarf an einer Lösung, das Intermediat 3-Methylthiopropionaldehyd möglichst auch in seiner z.B. destillativ erzeugten Reinform stabil lagern zu können. Auf den Einsatz kostenintensiver oder in späteren Prozessschritten störender Additive sollte dabei weitgehend verzichtet werden können. Auch sollte möglichst ohne Verwendung von zusätzlichen Gefahrstoffen eine Stabilisierung erreicht werden und die stabile Form möglichst direkt weiter zu Methionin umgesetzt werden können.

**Lösung**

Überraschenderweise wurde gefunden, dass die gezielte Zugabe von kleinen, z. B. substöchiometrischen Mengen an Wasser die Rückstandsbildung in destilliertem MMP deutlich vermindert. Die dabei erfindungsgemäß gebildete Verbindung gemäß Formel I, bei der durch ein Molekül Wasser zwei Moleküle MMP maskiert sind bewirkt, auch in der erfindungsgemäßen Zusammensetzung umfassend Verbindung gemäß Formel I, 3-Methylthiopropionaldehyd, 3-Methylthio-1,1-propandiol und Wasser, offenbar diesen sehr vorteilhaften Effekt. So wies beispielsweise destilliertes MMP mit 0,14 Gew.% Wassergehalt nach 6 Wochen Lagerung bei Raumtemperatur bereits 7,3 Gew.% Rückstand auf, während destilliertes MMP mit 10 Gew.% Wassergehalt nach 6 Wochen Lagerung nur 0,6 Gew.% Rückstand aufwies, also einen deutlich niedrigeren Wert (vgl. Beispiele 6 - 9, Figur 1). Auf diese Weise können die Verluste bei der Lagerung von destilliertem MMP in höchst einfacher und sehr kostengünstiger Weise vermieden werden.

Gegenstand der Erfindung ist daher eine chemische Verbindung der Formel (I), mit dem IUPAC-Namen 1-(1'-Hydroxy-3'-(methylthio)propoxy)-3-(methylthio)-propan-1-ol, deren erfindungsgemäße Bereitstellung zur Lösung der vorstehend genannten Aufgaben beiträgt.

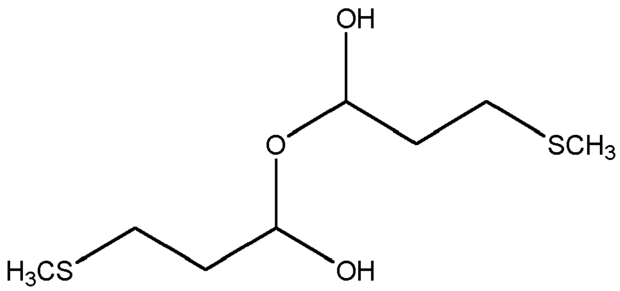

(I)

Die bislang noch nicht beschriebene Verbindung I kann als dimeres MMP-Hydrat aufgefasst werden ($MMP_2O$) und kommt in Form von zwei Diastereomeren vor, wobei ein Diastereomeres als Enantiomerenpaar vorliegt, das zweite als achirale meso-Form, mit den folgenden systematischen Namen:

- Meso-1-(1'-Hydroxy-3'-(methylthio)propoxy)-3-(methylthio)-propan-1-ol,
- 1-(1'R-1'-Hydroxy-3'-(methylthio)propoxy)-3-(methylthio)-propan-1S-ol und
- 1-(1'S-1'-Hydroxy-3-(methylthio)propoxy)-3-(methylthio)-propan-1R-ol.

Verbindung I kann vorteilhaft direkt in der herstellungsbedingt anfallenden Diastereomerenmischung erfindungsgemäß verwendet werden, jedoch auch in diastereomeren- oder enantiomerenreiner Form.

Ebenso kann das einfache MMP-Hydrat 3-Methylthio-1,1-propandiol als geschützte Form zur Lagerung und/oder zum Transport von 3-Methylthiopropionaldehyd verwendet werden. Diese Verbindung wurde erstmals beschrieben von C. Fernandez-Garcia in Chem. Commun. 2017, 53, 4919. Auf ihre erfindungsgemäße Verwendung gab es bisher jedoch keinen Hinweis.

Gegenstand der Erfindung ist daher auch eine Zusammensetzung umfassend 3-Methylthiopropionaldehyd, 3-Methylthio-1,1-propandiol, eine chemischen Verbindung gemäß Formel I und Wasser, durch welche die vorstehend genannten Aufgaben gelöst werden.

Bevorzugt wird dabei eine Zusammensetzung umfassend

40 bis 95 Gew.% 3-Methylthiopropionaldehyd,
1 bis 20 Gew.% 3-Methylthio-1,1-propandiol,
2 bis 25 Gew.% einer Verbindung gemäß Formel I und
2 bis 25 Gew.% Wasser.

Diese Zusammensetzung hat sich als besonders stabil und lagerfähig erwiesen.

Gegenstand der Erfindung ist somit auch die Verwendung von 3-Methylthio-1,1-propandiol oder einer Verbindung gemäß Formel I oder der vorstehend genannten Zusammensetzung als geschützte Form zur Lagerung und/oder zum Transport von 3-Methylthiopropionaldehyd. Die Lagerung erfolgt dabei vorzugsweise in einem Temperaturbereich von 0 bis 60 °C, besonders bevorzugt von 5 bis 40 °C in einem Zeitraum von bis zu 12 Monaten, vorzugsweise bis zu 8 Monaten und besonders bevorzugt bis zu 4 Monaten. Bei diesen Bedingungen wird die Rückstandsbildung auf einem niedrigen Niveau gehalten (vergleiche insbesondere Beispiele 8 und 9).

Die Zusammensetzung der vorstehend genannten Art kann erfindungsgemäß in vorteilhafter Weise hergestellt werden durch ein Verfahren, dadurch gekennzeichnet, dass 3-Methylthiopropionaldehyd mit 2,0 bis 25 Gew.% Wasser (bezogen auf die Gesamtmenge des Gemischs) unter intensiver Durchmischung umgesetzt wird bei einer Temperatur von 0 bis 100°C, vorzugsweise bei 3 bis 70°C, besonders bevorzugt bei 5 bis 20°C. Eine intensive Durchmischung der Komponenten wird in diesem Zusammenhang z.B. bei Einsatz eines Rührers mit ca. 60-180 Umdrehungen/min erreicht.

### Schema 1: Bildung von MMP-Hydraten

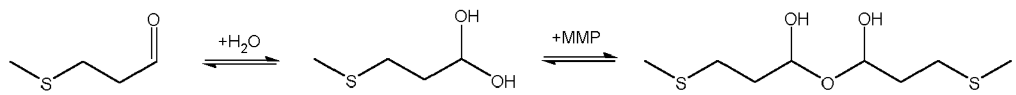


**MMP** ($C_4H_8OS$, 104.17 g/mol) **MMP-Hydrat** ($C_4H_{10}O_2S$, 122.18 g/mol) **dimeres MMP-Hydrat** ($C_8H_{18}O_3S_2$, 226.35 g/mol)

Dabei wird ein Verfahren bevorzugt, bei dem 3-Methylthiopropionaldehyd mit 2,0 bis 25 Gew.%, vorzugsweise mit 5,0 bis 20 Gew.% Wasser unter intensiver Durchmischung umgesetzt wird. Das erfindungsgemäße Verfahren zeichnen sich dadurch aus, dass es in sehr einfacher Weise ohne weitere Hilfsstoffe mit Wasser und bei moderaten Temperaturen erfolgreich durchgeführt werden kann.

Gegenstand der Erfindung ist damit auch die erfindungsgemäße Zusammensetzung umfassend 3-Methylthiopropionaldehyd, 3-Methylthio-1,1-propandiol, eine chemischen Verbindung gemäß Formel I und Wasser, welche durch ein erfindungsgemäßes Verfahren der vorher bezeichneten Art hergestellt worden ist.

Das vorstehend genannte erfindungsgemäße Verfahren eignet sich auch in sehr guter Weise zur Herstellung und Anreicherung bzw. Isolierung der Verbindung mit Formel I, welches in der Weise durchgeführt wird, dass 3- Methylthiopropionaldehyd mit 2,0 bis 30 Gew.% Wasser unter intensiver Durchmischung umgesetzt wird bei einer Temperatur von 0 bis 60°C, vorzugsweise bei 20 bis 50°C, ganz besonders bevorzugt bei 25 bis 45 °C und Verbindung I anschließend angereichert bzw. isoliert wird. Bei der Durchführung des erfindungsgemäßen Verfahrens aus technischem 3- Methylthiopropionaldehyd stellt sich typischerweise ein pH-Wert von 2,5 bis 7 in der Mischung ein, direkt gemessen mit einem pH-Meter mit Einstabmesskette. Bevorzugt liegt der pH-Wert im leicht sauren Bereich bei pH 3 bis 6. Er kann durch geringe Zugaben von organischer bzw. anorganischer Säure oder Base genau eingestellt werden. Dies ist insbesondere dann von Vorteil, wenn ganz bestimmte Gleichgewichtsanteile der genannten MMP-Derivate erhalten werden sollen.

Die Anreicherung bzw. Isolierung der Verbindung der Formel I in Reinform erfolgt zweckmäßigerweise dann mittels Chromatographie, Kristallisation oder Vakuumdestillation. Da die Verbindung der Formel I im temperatur-abhängigen Gleichgewicht mit MMP, dem MMP-Hydrat und Wasser vorliegt, werden zweckmäßigerweise nur schonende Verfahrensvarianten verwendet. Dabei sind beim Verfahren der Chromatographie solche Trägerstoffe als stationäre Phase auszuwählen, die einen basischen bis neutralen pH-Wert ergeben. Analoges gilt für das Laufmittel, das keine saure Reaktion zeigen darf. Für die destillative Aufreinigung kommen insbesondere Dünnfilmverdampfer- und Kurzwegverdampfersysteme in Frage, die besonders schonende Bedingungen im Hochvakuum ermöglichen. Bei allen erfindungsgemäßen Verfahrensvarianten wird bevorzugt eine Rein-MMP Qualität eingesetzt, also ein 3-Methylthiopropionaldehyd, der zuvor beispielsweise destilliert worden ist.

Sowohl 3-Methylthio-1,1-propandiol, als auch die Verbindung I oder eine Zusammensetzung der vorstehend genannten Art können direkt verwendet werden, anstelle von MMP zur Herstellung von 2-Hydroxy-4-(methylthio)buttersäurenitril (MMP-Cyanhydrin), einem industriell sehr wichtigen Zwischenprodukt auf dem Wege zum Methionin bzw. auch zum Methioninhydroxyanalogen. Dabei werden die genannten Ausgangsstoffe mit jeweils äquimolaren Mengen Blausäure umgesetzt, wobei die Blausäure auch erst in situ aus einem ihrer Salze freigesetzt werden kann, wie beispielsweise aus Alkalimetallcyanid und einem protonenspendenden Salz wie z.B. $NH_4Cl$, $NH_4HSO_4$ oder $(NH_4)_2SO_4$.

Sowohl 3-Methylthio-1,1-propandiol als auch die Verbindung I oder eine Zusammensetzung der vorstehend genannten Art können direkt verwendet werden anstelle von MMP zur Herstellung von Methioninhydantoin, dem zentralen Zwischenprodukt einer gängigen Methionin Synthese, dem Degussa-Kaliumcarbonat-Kreislauf Verfahren. Die Umsetzung erfolgt dabei beispielsweise direkt mit Blausäure, Ammoniak und Kohlendioxid bzw. Ammoniumhydrogencarbonat/-carbonat oder über die zuvor durch Umsetzung mit Blausäure erzeugte Zwischenstufe des 2-Hydroxy-4-(methylthio)buttersäurenitrils.

Desweiteren erfindungsgemäß entsprechend verwendbar sind 3-Methylthio-1,1-propandiol oder Verbindung I oder eine Zusammensetzung der vorstehend genannten Art anstelle von MMP zur Herstellung von Methionin, z.B. über die Zwischenstufe des Methioninhydantoin.

Ebenso Gegenstand der vorliegenden Erfindung ist die Verwendung von 3-Methylthio-1,1-propandiol oder einer Verbindung gemäß der Formel I oder einer Zusammensetzung der vorstehend genannten Art zur Herstellung des Methioninersatzstoffs 2- Hydroxy-4-(methylthio)buttersäure (MHA) in dem man zunächst wie bereits vorstehend ausgeführt die MHA-Vorstufe 2-Hydroxy-4-(methylthio)buttersäurenitril daraus herstellt. Die erfindungsgemäße Verwendung kann auf diese Weise z.B. in eine wie in EP 143100 B1 (insbesondere Beispiel 1) beschriebene Verfahrensweise zur MHA-Herstellung integriert werden.

Entsprechende weitere Gegenstände der vorliegenden Erfindung sind ein Verfahren zur Herstellung von 2-Hydroxy-4-(methylthio)buttersäurenitril, dadurch gekennzeichnet, dass man 3-Methylthio-1,1-propandiol oder eine Verbindung gemäß Anspruch 1 oder eine oben aufgeführte erfindungsgemäße Zusammensetzung in Gegenwart einer Aminbase (z.B. Triethylamin, Pyridin oder Lutidin) und ggf. zusätzlich einer Säure (Puffer) mit Blausäure oder anderen Cyanidquellen umsetzt. Die Verfahrensparameter können ansonsten denen entsprechen, die der Fachmann für Verfahren ausgehend von MMP aus den Beschreibungen in der einschlägigen Fachliteratur kennt, wie beispielsweise der EP 2678313 A1 (insbesondere Beispiele 1 und 2) oder der EP 2768312 A1 sowie der US 5705675 (inbesondere Beispiel 4, Spalte 13, ab Zeile 29) und der darin jeweils zitierten einschlägigen Literatur, welche allesamt hiermit per Referenz einbezogen sind.

Ebenso Gegenstand der vorliegenden Erfindung ist ein Verfahren zur Herstellung von Methioninhydantoin dadurch gekennzeichnet, dass man 3-Methylthio-1,1-propandiol oder eine Verbindung gemäß Formel I oder eine Zusammensetzung der vorstehend bezeichneten Art mit Blausäure, Ammoniak und Kohlendioxid bzw. Ammonium-hydrogencarbonat/-carbonat oder über die zuvor durch Umsetzung mit Blausäure erzeugte Zwischenstufe des 2-Hydroxy-4-(methylthio)buttersäurenitrils in Gegenwart von Wasser umsetzt. Die Verfahrensparameter können ansonsten denen entsprechen, die der Fachmann für Verfahren ausgehend von MMP aus den Beschreibungen in der einschlägigen Fachliteratur kennt, wie beispielsweise der EP 780370 A2 (insbesondere Beispiel 1 bis 4) und der darin jeweils zitierten einschlägigen Literatur, welche allesamt hiermit per Referenz einbezogen sind.

Schließlich ist ein weiterer Gegenstand der vorliegenden Erfindung ein entsprechendes Verfahren zur Herstellung von D,L-Methionin durch Umsetzung einer Zusammensetzung der vorstehend bezeichneten Art mit HCN, $NH_3$, Kohlendioxid zum Methioninhydantoin und anschließende alkalische Verseifung zum Methionin-Alkalisalz und nachfolgende Neutralisation mit Säure zum Methionin. Auch die zu verwendenden Verfahrensparameter kann der Fachmann aus der einschlägigen Fachliteratur wie beispielsweise der EP 780370 A2 entnehmen, die hiermit per Referenz einbezogen wird, insbesondere die Beispiele 1 bis 7.

Die entsprechenden erfindungsgemäßen Beispiele 11 und 12 zeigen hohe Umsätze und Gesamtausbeuten an Methionin, Methioninamid und Methionylmethionin.

Die diesbezüglich in Tabelle 4 zusammengefassten Ergebnisse machen auch deutlich, dass die $MMP_2O$- reichen erfindungsgemäßen Zusammensetzungen mit einem Wassergehalt $\geq$ 2 Gew.% (Beispiele 11, 12) und die $MMP_2O$- armen nicht erfindungsgemäßen Zusammensetzungen mit einem Wassergehalt von nur 1 Gew.% (Vergleichsbeispiele 1, 2) bei der Herstellung von Methionin vergleichbare Ausbeuten und Nebenproduktzusammensetzungen liefern.

Diese Ergebnisse zeigen auch, dass die in der erfindungsgemäßen Zusammensetzung in Anteilen von z.T. deutlich über 2 Gew.% enthaltenen MMP-Derivate $MMP_2O$ und MMP-Hydrat unter vergleichbaren Ausführungsbedingungen überraschenderweise zumindest vergleichbar gut zu Methioninäquivalenten im Endprodukt umgesetzt werden können wie nicht derivatisiertes MMP.

## Beispiele

### Verwendete Methoden:

#### 1. Rückstandsbestimmung mittels Vakuum-Destillation

Die Rückstandsbestimmung wurde in einem Kugelrohrverdampfer des Typs GKR-50 der Firma Büchi durchgeführt. Hierzu wurde zunächst das Leergewicht der für die Destillation eingesetzten Vorlage bestimmt. Dann wurde eine Gewichtsmenge von 15 g der zu destillierenden Substanz (m (Einwaage)) genau eingewogen und die Vorlage in den Kugelrohrverdampfer eingebracht. Die Heizung der Destillationsvorlage wurde auf 200°C eingestellt, und über den Druckregler der Vakuumpumpe wurde ein Druck von 30 mbar eingestellt. Bei allen Proben erfolgte die Destillation über einen Zeitraum von 20 min. Nach dem Abkühlen der Destillationsapparatur wurde die Apparatur belüftet. Anschließend

wurde die Vorlage entfernt und gewogen zur Bestimmung der Gewichtsmenge (m (Rückstand)). Der Rückstand wurde mit der folgenden Formel bestimmt:

$$\text{Rückstand [Gew.} - \%] = \frac{\text{m (Rückstand)}}{\text{m (Einwaage)}}$$

**2. NMR-spektroskopische Untersuchungen**

Der Gehalt der im Gleichgewicht vorliegenden Verbindungen 3-Methylthiopropionaldehyd, 3-Methylthio-1,1-propandiol und 1-(1'-Hydroxy-3-(methylthio)propoxy)-3-(methylthio)-propan-1-ol in einer Probe wurde mittels NMR-Spektroskopie (nuclear magentic resonance) an einem Gerät des Typs Advance 600 von Bruker bestimmt.

Für die Beispiele wurden $^{13}$C-NMR-Spektren von Proben, die nicht mit einem Lösungsmittel versetzt waren, bei 150 MHz aufgenommen. Aus den aufgenommenen Spektren wurden die molaren Verhältnisse der Bestandteile zueinander abgelesen. Als Referenzsubstanz diente $d_6$-DMSO, das in einer zugeschmolzenen Kapillare in das NMR-Röhrchen der betreffenden Probe eingebracht wurde. Zur Verhältnisbildung wurden charakteristische Signale von 3-Methylthiopropionaldehyd, 3-Methylthio-1,1-propandiol und 1-(1'-Hydroxy-3-(methylthio)propoxy)-3-(methylthio)-propan-1-ol im $^{13}$C-NMR-Spektrum ausgewählt. Dies waren für 3-Methylthiopropionaldehyd das Signal $-\underline{C}H_2$-CHO bei 42.3 ppm, für 3-Methylthio-1,1-propandiol das Signal $-CH_2-\underline{C}(OH)_2$ bei 89.7 ppm und für 1-(1'-Hydroxy-3-(methylthio)propoxy)-3-(methylthio)-propan-1-ol das Signal $O(\underline{C}(OH)-CH_2-CH_2-SCH_3)_2$ bei 91.2 bzw. 96.4 ppm, hier wurde das Summenintegral über beide Diastereomere gebildet.

Die Berechnung der Massenverhältnisse erfolgte aus den molaren Verhältnissen unter Berücksichtigung des mittels Karl-Fischer Titration bestimmten Gesamtwassergehalts.

Die angehängten Figuren 2 und 3 zeigen jeweils ein exemplarisches $^{13}$C-NMR (150 MHz, ohne Lösungsmittel, $d_6$-DMSO-Kapillare) und 1H-NMR Spektrum (600 MHz, $d_6$-DMSO) einer erfindungsgemäßen Zusammensetzung.

**3. Bestimmung des Wassergehalts mittels Karl-Fischer-Titration**

Der Gehalt an Wasser in den erfindungsgemäßen Zusammensetzungen wurde nach der Methode von Karl Fischer durch Titration mit biamperometrischer Indikation des Endpunkts bestimmt. Hierzu wurden im Titriergefäß 20 bis 30 ml Titriermedium, z.B. Hydranal Solvent 5 der Firma Fluka, vorgelegt und mit Titriermittel, z.B. Hydranal Titrant 5 der Firma Fluka, trockentitriert. Eine Probenmenge von ca. 500 mg wurde der trockentitrierten Vorlage mit einer Kunststoffeinwegspritze zugesetzt und mit dem Titriermittel bis zum Endpunkt titriert. Die Bestimmung der genauen Probeneinwaage erfolgte durch Differenzwägung.

Die Durchführung dieser Standardmethode ist dem Fachmann bekannt und ausgiebig in der einschlägigen Literatur beschrieben, beispielsweise in P. A. Bruttel, R. Schlink, "Wasserbestimmung durch Karl-Fischer-Titration", Metrohm AG, 2006).

**4. High Performance Liquid Chromatography (HPLC)**

Die chromatographischen Untersuchungen (MMP-Cyanhydrin, MMP, Methionin, Methioninamid, Hydantoin, Hydantoinamid, Hydantoinsäure, Met-Met) wurden mittels HPLC der Firma JASCO an einer RP-18 Säule (250 x 4,6 mm; 5 $\mu$m) mit anschließender UV Detektion bei 210 nm durchgeführt. Als Laufmittel diente ein phosphorsaures Acetonitril-Wasser-Gemisch (3,3 g $H_3PO_4$, 6,8 g Acetonitril, 89,9 g $H_2O$). Bei einem Fluss von 1 mL/min wurden 10 $\mu$L der jeweiligen Probelösung (50 mg Probe in 25 mL $H_2O$) injiziert. Die Kalibrierung erfolgte im Vorfeld durch die Injektion geeigneter Kalibrierlösungen, die Auswertung erfolgte durch Peakflächenvergleich mittels der externen Standardmethode. Die Durchführung der Standardmethode ist dem Fachmann bekannt.

**Beispiele 1-2: Herstellung von 3-Methylthio-1,1-propandiol (MMP-OH) und von 1-(1'-Hydroxy-3-(methylthio)propoxy)-3-(methylthio)-propan-1-ol ($MMP_2O$)**

Destilliertes 3-Methylthiopropionaldehyd aus industrieller Herstellung (97,4 Gew%; 196,0 g (Beispiel 1), 180,1 g (Beispiel 2)) wurde in einem Kolben vorgelegt und unter Rühren bei Raumtemperatur mit Wasser (4,0 g (Beispiel 1), 20,0 g (Beispiel 2)) versetzt. Anschließend wurde die erhaltene Produktmischung für 3 Tage bei Raumtemperatur und mittels NMR-Spektroskopie und Karl-Fischer-Titration analysiert. In Beispiel 1 wurde ein Gehalt an $MMP_2O$ von 2,9 mol% (entsprechend 6,0 Gew%, vgl. Tab.1) ermittelt. In Beispiel 2 wurde ein Gehalt von 7,9 mol% bzw. 14,3 Gew% ermittelt. Die Ergebnisse der Beispiele 1 und 2 sind in Tabelle 1 zusammengefasst.

**Tabelle 1: Übersicht über die Beispiele 1 und 2**

| Beispiel | Edukte | | Produktzusammensetzung | | | | | | |
|---|---|---|---|---|---|---|---|---|---|
| | MMP | $H_2O$ | MMP | | MMP-OH | | $MMP_2O$ | | $H_2O$ |
| | Gew/Gew | | mol%* | Gew%** | mol%* | Gew%** | mol%* | Gew%** | Gew%** |
| 1 | 98 | 2 | 95,1 | 90,6 | 2,0 | 2,2 | 2,9 | 6,0 | 1,2 |
| 2 | 90 | 10 | 81,8 | 68,2 | 10,3 | 10,1 | 7,9 | 14,3 | 7,4 |
| * Relative mol-Verhältnisse aus dem 13-C NMR ohne Berücksichtigung des freien Wassers.<br>** Berechnete Gew% unter Berücksichtigung des zugefügten Wasseranteils | | | | | | | | | |

**(MMP = 3-Methylthiopropionaldehyd, MMP-OH = 3-Methylthio-1,1-propandiol, $MMP_2O$ = 1-(1'-Hydroxy-3-(methylthio)propoxy)-3-(methylthio)-propanol)**

**Beispiele 3-5: Untersuchung der Gleichgewichtslage einphasiger wassergesättigter Gemische mit erfindungsgemäßer Zusammensetzung in Abhängigkeit der Temperatur**

Destilliertes 3-Methylthiopropionaldehyd aus industrieller Herstellung (96,1 Gew%; jeweils 30 mL) wurde in einem Reaktionskolben mit Temperiermantel mit Wasser (jeweils 30 mL) versetzt. Die Mischung wurde temperiert (Beispiel 3: 5 °C, Beispiel 4: 25 °C, Beispiel 5: 40 °C) und 15 min bei dieser Temperatur unter starkem Rühren (120 U/min) vermischt. Nach 15 min wurde der Rührer ausgeschaltet, die Temperierung wurde weiterhin beibehalten. Innerhalb weniger Minuten (Beispiel 5) bzw. einiger Tage (Beispiel 3) bildeten sich zwei klare, farblose Phasen. Die untere organische Phase wurde entnommen und zügig mittels HPLC, temperierter NMR Spektroskopie sowie Karl-Fischer Titration charakterisiert. Der höchste Gehalt an $MMP_2O$ wurde mit ca. 23 Gew% bei 25 °C ermittelt (Beispiel 4). Den höchsten Gehalt an MMP-OH innerhalb der Versuchsreihe wies die Probe bei 5 °C auf, es wurde ein Gehalt von ca. 15 Gew% ermittelt (Beispiel 3). Die Ergebnisse der Beispiele 3 bis 5 sind in Tabelle 2 zusammengefasst.

**Tabelle 2: Übersicht über die Beispiele 3 bis 5 wassergesättigter Mischungen der erfindungsgemäßen Zusammensetzung bei verschiedenen Temperaturen**

| Beispiel | Temperatur | Produktzusammensetzung* | | | |
|---|---|---|---|---|---|
| | | MMP | MMP-OH | $MMP_2O$ | $H_2O$ |
| | | (Gew%) | (Gew%) | (Gew%) | (Gew%) |
| 3 | 5 °C | 45,0 | 15,3 | 17,9 | 21,0 |
| 4 | 25 °C | 51,8 | 12,2 | 23,2 | 12,5 |
| 5 | 40 °C | 63,1 | 4,9 | 21,0 | 11,0 |
| * Rechnerisch bestimmt mittels HPLC (Gesamtgehalt MMP) und Karl-Fischer-Analyse (Gesamtgehalt $H_2O$) sowie $^{13}C$-NMR Spektroskopie (molare Verhältnisse MMP : MMP-OH : $MMP_2O$). | | | | | |

**(MMP = 3-Methylthiopropionaldehyd, MMP-OH = 3-Methylthio-1,1-propandiol, $MMP_2O$ = 1-(1'-Hydroxy-3-(methylthio)propoxy)-3-(methylthio)-propanol)**

**Beispiele 6-9: Lagerstabilität von 1-(1'-Hydroxy-3'-(methylthio)propoxy)-3-(methylthio)-propanol ($MMP_2O$) enthaltenden Gemischen**

Destilliertes 3-Methylthiopropionaldehyd (MMP, 99,8 Gew%; Wassergehalt 0,14 Gew%) wurde unter Rühren bei Raumtemperatur mit verschiedenen Mengen an Wasser versetzt (Beispiel 6: 0,0 g Wasser auf 182,2 g MMP; Beispiel 7: 4,6 g Wasser auf 179,4 g MMP; Beispiel 8: 9,4 g Wasser auf 178,6 g MMP; Beispiel 9: 19,6 g Wasser auf 176,4 g MMP). Die Proben wurden über mehrere Wochen bei Raumtemperatur gelagert und in regelmäßigen Zeitabständen wurde der Rückstand mittels Vakuumdestillation bestimmt. Die Ergebnisse sind in Tabelle 3 sowie der angehängten Abbildung 1 zusammengefasst. Die Ergebnisse zeigen deutlich, dass eine Mischung mit erhöhtem Anteil an $MMP_2O$ wie insbesondere bei Beispiel 8 und 9 eine deutlich verringerte Rückstandsbildung als eine Mischung ohne $MMP_2O$ aufweist.

**Tabelle 3: Übersicht über die Beispiele 6 bis 9**

| **Beispiel** | **6** | **7** | **8** | **9** |
|---|---|---|---|---|
| $H_2O$ in MMP | 0 Gew% | 2,5 Gew% | 5 Gew% | 10 Gew% |
| $MMP_2O$-Gehalt* | 0 Gew% | 5 Gew% | 8 Gew% | 15 Gew% |
| **Zeit in Wochen** | **Rückstand in Gew%** | | | |
| 0 | 0,00 | 0,02 | 0,05 | 0,00 |
| 2 | 1,35 | 1,03 | 0,48 | 0,27 |
| 4 | 3,39 | 2,18 | 0,26 | 0,40 |
| 6 | 7,37 | 3,51 | 0,90 | 0,60 |
| 8 | 9,06 | 5,01 | 1,10 | 0,37 |
| * abgeschätzte Werte aus NMR Spektren. | | | | |

**(MMP = 3-Methylthiopropionaldehyd, $MMP_2O$ = 1-(1'-Hydroxy-3-(methylthio)propoxy)-3-(methylthio)-propan-1-ol)**

**Beispiel 10: Herstellung von 2-Hydroxy-4-(methylthio)buttersäurenitril (MMP-Cyanhydrin) aus einer erfindungsgemäßen 1-(1'-Hydroxy-3-(methylthio)propoxy)-3-(methylthio)-propan-1-ol ($MMP_2O$), 3-Methylthio-1,1-propandiol (MMP-OH = MMP-Hydrat) und MMP enthaltenden Zusammensetzung**

180,1 g destilliertes 3-Methylthiopropionaldehyd aus industrieller Herstellung (97,4 Gew%) wurde in einem Kolben vorgelegt und unter Rühren bei Raumtemperatur mit 20,0 g Wasser versetzt. Anschließend wurde die erhaltene Produktmischung für 3 Tage bei Raumtemperatur nachgerührt. Eine Charakterisierung per NMR-Spektroskopie und Karl-Fischer Titration ergab einen Gehalt von 68,2 Gew% 3-Methylmercaptopropionaldehyd, 10,1 Gew% MMP-OH und 14,3 Gew% $MMP_2O$ (nachfolgend als MMP-Äquivalente bezeichnet).

173,6 g der Produktmischung (1,50 mol MMP-Äquivalente) wurde in einem mit Intensivkühler und Tropftrichter bestücktem Dreihalskolben mit katalytischen Mengen Triethanolamin (58 mg) versetzt und mittels Eisbad gekühlt. Über den Tropftrichter wurde über einen Zeitraum von 20 min gekühlte Blausäure (43,48 g, 1,59 mol, 1,06 Äquiv.) hinzugefügt. Die Dosierung wurde dabei so reguliert, das eine Temperatur von 30 °C nicht überschritten wurde. Die Lösung wurde über Nacht bei Raumtemperatur nachgerührt. Das farblose, klare Produkt (205,28 g) wurde mittels HPLC-Analyse charakterisiert und enthielt 92,82 Gew% der Zielverbindung MMP-Cyanhydrin (1,45 mol, 96,8% Ausbeute bezogen auf eingesetzte MMP-Äquivalente) sowie 1,15 Gew% an mit freier Blausäure im Gleichgewicht stehendem 3-Methylmercaptopropionaldehyd (0,03 mol, 2 Gew%). Die vorstehenden Ergebnisse zeigen, dass die Herstellung von MMP-Cyanhydrin auch aus mit $MMP_2O$ und MMP-OH angereichertem, wasserhaltigem MMP, also der erfindungsgmäßen Zusammensetzung, erfolgen kann.

**Beispiele 11-12 mit Vergleichsbeispielen 1-2: Herstellung von Methionin über die Zwischenstufen 5-[2'-(Methylthio)ethyl]-imidazolidin-2,4-dion (Methioninhydantoin) und MMP-Cyanhydrin aus einer erfindungsgemäßen 1-(1'-Hydroxy-3-(methylthio)propoxy)-3-(methylthio)-propan-1-ol ($MMP_2O$) und MMP enthaltenden Zusammensetzung**

180,1 g destilliertes 3-Methylthiopropionaldehyd aus industrieller Herstellung (97,4 Gew%) wurde in einem Kolben vorgelegt und unter Rühren bei Raumtemperatur mit 20,0 g Wasser versetzt. Anschließend wurde die erhaltene Produktmischung für 3 Tage bei Raumtemperatur nachgerührt. Eine Charakterisierung per NMR-Spektroskopie und Karl-Fischer Titration ergab einen Gehalt von 68,2 Gew% 3-Methylmercaptopropionaldehyd, 10,1 Gew% MMP-OH, 14,3 Gew% $MMP_2O$ und 7,4 Gew.% Wasser.

173,6 g der Produktmischung (1,50 mol MMP-Äquivalente) wurde in einem mit Intensivkühler und Tropftrichter bestücktem Dreihalskolben mit katalytischen Mengen Triethanolamin (58 mg) versetzt und mittels Eisbad gekühlt. Über den Tropftrichter wurde über einen Zeitraum von 20 min gekühlte Blausäure (43,48 g, 1,59 mol, 1,06 Äquiv.) hinzugefügt. Die Dosierung wurde dabei so reguliert, dass eine Temperatur von 30 °C nicht überschritten wurde. Die Lösung wurde über Nacht bei Raumtemperatur nachgerührt und der Blausäureüberschuss mittel Charakterisierung bestimmt. 12,6 g an 3-Methylmercaptopropionaldehyd (0,12 mol) wurden nachdosiert und für 20 h bei Raumtemperatur nachgerührt, um den Blausäureüberschuss auf 0.0 mol% zu regulieren.

37,2 g des so erhaltenen MMP-Cyanhydrins (0,257 mol an MMP-Äquivalenten) wurde in einem mit einem Rührfisch bestückten 300 mL Autoklavbecher mit destilliertem Wasser (35,5 g), Ammoniumcarbonat (13,9 g, 0,15 mol) und Ammoniumhydrogencarbonat (23,4 g, 0,30 mol) versetzt. Das Reaktionsgefäß wurde in einen Hochdruck-Laborautoklaven der Firma ROTH, bestückt mit Manometer, Heizung, Temperaturfühler und Druckablass, überführt. Der Autoklav wurde fest verschlossen, unter Rühren innerhalb von 15 min auf 105 °C aufgeheizt und dann weitere 20 min auf dieser Temperatur gehalten. Nach Ablauf der Reaktionszeit wurde der Autoklav unter fließendem Wasser auf Raumtemperatur abgekühlt und der entstandene Druck (ca. 15 bar) abgelassen.

Nun wurde über das Einleitrohr 41,3 g wässrige KOH-Lösung (17 g KOH in 24,3 g $H_2O$) zudosiert. Nach beendigter Zugabe wurde der Autoklav unter Rühren innerhalb von 25 min auf 180 °C geheizt und dann weitere 40 min auf dieser Temperatur gehalten. Während der Reaktionszeit wurde der Druck ca. alle 5 min, mindestens aber bei Überschreitung von 10 bar, auf 5 bar abgelassen. Nach Ablauf der Reaktionszeit wurde der Autoklav unter fließendem Wasser auf Raumtemperatur abgekühlt und auf Normdruck entspannt. HPLC-Analyse des Reaktionsproduktes (134,7 g) ergab bei 97,2% Umsatz 60,1% Methionin, 6,9% Methioninamid und 30,2% Methionylmethionin. In einem Wiederholungsversuch (Beispiel 12) wurden bei 99,5% Umsatz 71,4% Methionin, 6,6% Methioninamid und 21,4% Methionylmethionin erhalten.

**Vergleichsbeispiele 1 und 2**

Ein Vergleichsbeispiel 1 zur Herstellung von Methionin analog Beispiel 11 aber mit aus $MMP_2O$-armem 3-Methylmercaptopropionaldehyd (enthaltend 1,2 Gew% Wasser) nach Beispiel 1 hergestelltem MMP-Cyanhydrin ergab bei 95,0% Umsatz 63,0% Methionin, 5,5% Methioninamid und 26,5% Methionylmethionin; in einem Wiederholungsversuch (Vergleichsbeispiel 2) wurden bei 96,5% Umsatz 65,8% Methionin, 6,5% Methioninamid und 24,2% Methionylmethionin erhalten.

**Tabelle 4: Gegenüberstellung der Methionin-Herstellung aus $MMP_2O$-reicher und $MMP_2O$-armer MMP-haltiger erfindungsgemäßer Zusammensetzung**

| | **Eduktzusammensetzung, Gew%** | **Umsatz, %** |
|---|---|---|
| | **$MMP_2O$ (MMP, MMP-OH, $H_2O$)** | **Methionin (Met-Amid; Met-Met)** |
| Beispiel 11 | 14,3 (68,2; 10,1; 7,4) | 60,1 (6,9; 30,2) |
| Beispiel 12 | | 71,4 (6,6; 21,4) |
| Vergleichsbeispiel 1 | 6,0 (90,6; 2,2; 1,2) | 63,0 (5,5; 26,5) |
| Vergleichsbeispiel 2 | | 65,8 (6,5: 24,2) |

**(MMP = 3-Methylthiopropionaldehyd, MMP-OH = 3-Methylthio-1,1-propandiol, $MMP_2O$ = 1-(1'-Hydroxy-3-(methylthio)propoxy)-3-(methylthio)-propan-1-ol)**

Die in Tabelle 4 zusammengefassten Ergebnisse zeigen, dass $MMP_2O$- reiche erfindungsgemäße Zusammensetzungen (Beispiele 11, 12) und $MMP_2O$- arme erfindungsgemäße Zusammensetzungen (Vergleichsbeispiele 1, 2) bei der Herstellung von Methionin vergleichbare Ausbeuten und Nebenproduktspektren liefern, sodass offenbar auch $MMP_2O$ (1-(1'-Hydroxy-3-(methylthio)propoxy)-3-(methylthio)-propan-1-ol) sowie MMP-OH (3-Methylthiopropan-1,1-diol) über MMP-Cyanhydrin und Methionin-Hydantoin zum Methionin umgesetzt werden. Die relativ hohen Anteile an Met-Met von über 20 % sind dabei auf die labortechnische Ausführung zurückzuführen und liegen in kontinuierlichen technischen Verseifungskolonnen des Methioninverfahrens im niedrigen Prozentbereich.

## Patentansprüche

1. Chemische Verbindung der Formel (I)

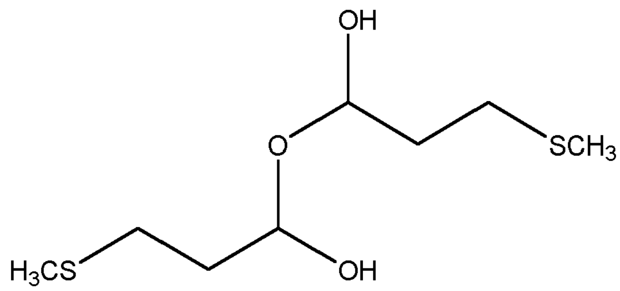

.

**2.** Zusammensetzung umfassend 3-Methylthiopropionaldehyd, 3-Methylthio-1,1-propandiol, eine Verbindung gemäß Anspruch 1 und Wasser.

**3.** Zusammensetzung gemäß Anspruch 2, umfassend

40 bis 95 Gew.% 3-Methylthiopropionaldehyd,
1 bis 20 Gew.% 3-Methylthio-1,1-propandiol,
2 bis 25 Gew.% einer Verbindung der Formel I gemäß Anspruch 1 und
2 bis 25 Gew.% Wasser.

**4.** Verwendung von 3-Methylthio-1,1-propandiol oder einer Verbindung gemäß Anspruch 1 oder einer Zusammensetzung gemäß einem der Ansprüche 2 bis 3 zur Lagerung und/oder zum Transport von 3-Methylthiopropionaldehyd.

**5.** Verwendung gemäß Anspruch 4, **dadurch gekennzeichnet, dass** die Lagerung und /oder der Transport bei Temperaturen zwischen 0 und 60 °C, vorzugsweise 5 bis 40 °C durchgeführt werden in einem Zeitraum von bis zu 12 Monaten, vorzugsweise bis zu 8 Monaten besonders bevorzugt bis zu 4 Monaten.

**6.** Verwendung von 3-Methylthio-1,1-propandiol oder einer Verbindung gemäß Anspruch 1 oder 2 oder einer Zusammensetzung gemäß einem der Ansprüche 2 bis 3 zur Herstellung von 2-Hydroxy-4-(methylthio)buttersäurenitril.

**7.** Verwendung von 3-Methylthio-1,1-propandiol oder einer Verbindung der Formel I gemäß Anspruch 1 oder einer Zusammensetzung gemäß einem der Ansprüche 2 bis 3 zur Herstellung von Methioninhydantoin.

**8.** Verwendung von 3-Methylthio-1,1-propandiol oder einer Verbindung der Formel I gemäß Anspruch 1 oder einer Zusammensetzung gemäß einem der Ansprüche 2 bis 3 zur Herstellung von Methionin.

**9.** Verwendung von 3-Methylthio-1,1-propandiol oder einer Verbindung der Formel I gemäß Anspruch 1 oder einer Zusammensetzung gemäß einem der Ansprüche 2 bis 3 zur Herstellung von 2-Hydroxy-4-(methylthio)buttersäure.

**10.** Verfahren zur Herstellung einer Zusammensetzung gemäß einem der Ansprüche 2 bis 3, **dadurch gekennzeichnet, dass** 3-Methylthiopropionaldehyd mit 2,0 bis 25 Gew.%, vorzugsweise mit 5,0 bis 20 Gew.% Wasser bezogen auf die Gesamtmenge an eingesetztem 3-Methylthiopropionaldehyd und Wasser unter intensiver Durchmischung umgesetzt wird bei einer Temperatur von 0 bis 100°C, vorzugsweise bei 3 bis 70°C, ganz besonders bevorzugt bei 5 bis 40°C.

**11.** Verfahren gemäß Anspruch 10, **dadurch gekennzeichnet, dass** 3-Methylthiopropionaldehyd mit 2,0 bis 25 Gew.% Wasser unter intensiver Durchmischung umgesetzt wird bei einer Temperatur von 0 bis 100°C und Verbindung I anschließend isoliert oder zumindest angereichert wird, vorzugsweise mittels Chromatographie, Kristallisation oder Vakuumdestillation.

**12.** Verfahren gemäß einem der Ansprüche 10 bis 11, **dadurch gekennzeichnet, dass** der eingesetzte 3-Methylthiopropionaldehyd zuvor destilliert worden ist.

**13.** Verfahren zur Herstellung von 2-Hydroxy-4-(methylthio)buttersäurenitril, **dadurch gekennzeichnet, dass** man 3-Methylthio-1,1-propandiol oder einer Verbindung gemäß Anspruch 1 oder eine Zusammensetzung gemäß einem der Ansprüche 2 bis 3 in Anwesenheit einer Aminbase mit Blausäure umsetzt.

**14.** Verfahren zur Herstellung von Methioninhydantoin **dadurch gekennzeichnet, dass** man 3-Methylthio-1,1-propandiol oder eine Verbindung gemäß Anspruch 1 oder eine Zusammensetzung gemäß einem der Ansprüche 2 bis 3 mit Blausäure, Ammoniak und Kohlendioxid in Gegenwart von Wasser umsetzt.

**15.** Verfahren zur Herstellung von D,L-Methionin durch Umsetzung einer Zusammensetzung gemäß Anspruch 2 oder 3 mit HCN, $NH_3$, Kohlendioxid zum Methioninhydantoin und anschließende alkalische Verseifung zum Methionin-Alkalisalz und nachfolgende Neutralisation mit Säure zum Methionin.

**Figur 1:** **Trends der Rückstandsbildung in 3-Methylmercaptopropionaldehyd mit variiertem Wassergehalt (Beispiele 6-9)**

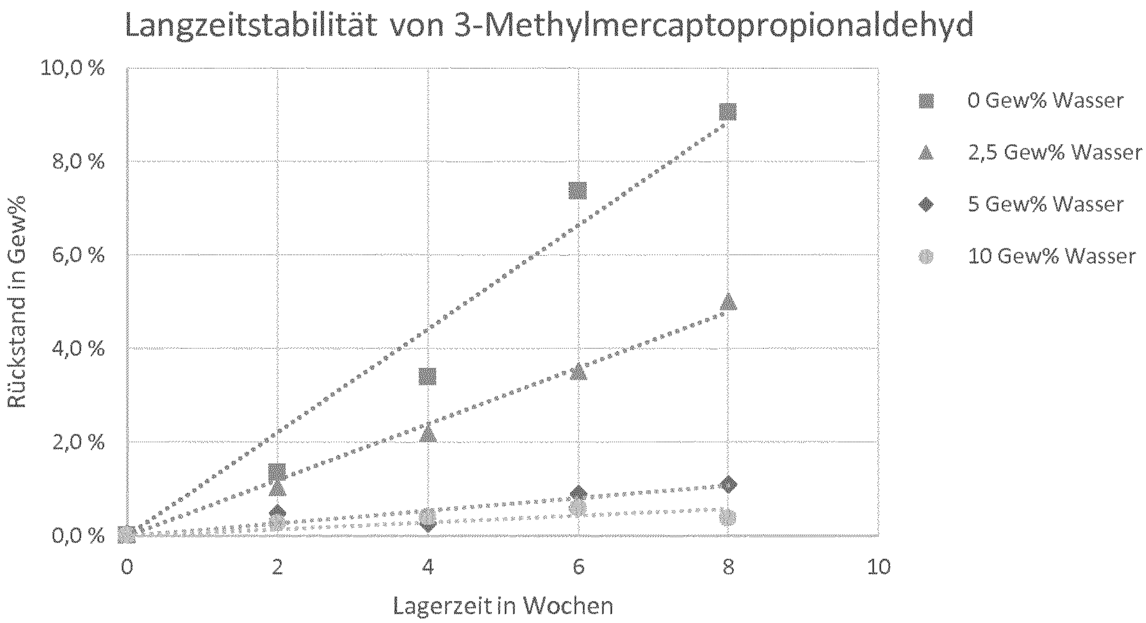

Figur 2: **Beispielhaftes 13C-NMR-Spektrum einer erfindungsgemäßen Zusammensetzung**

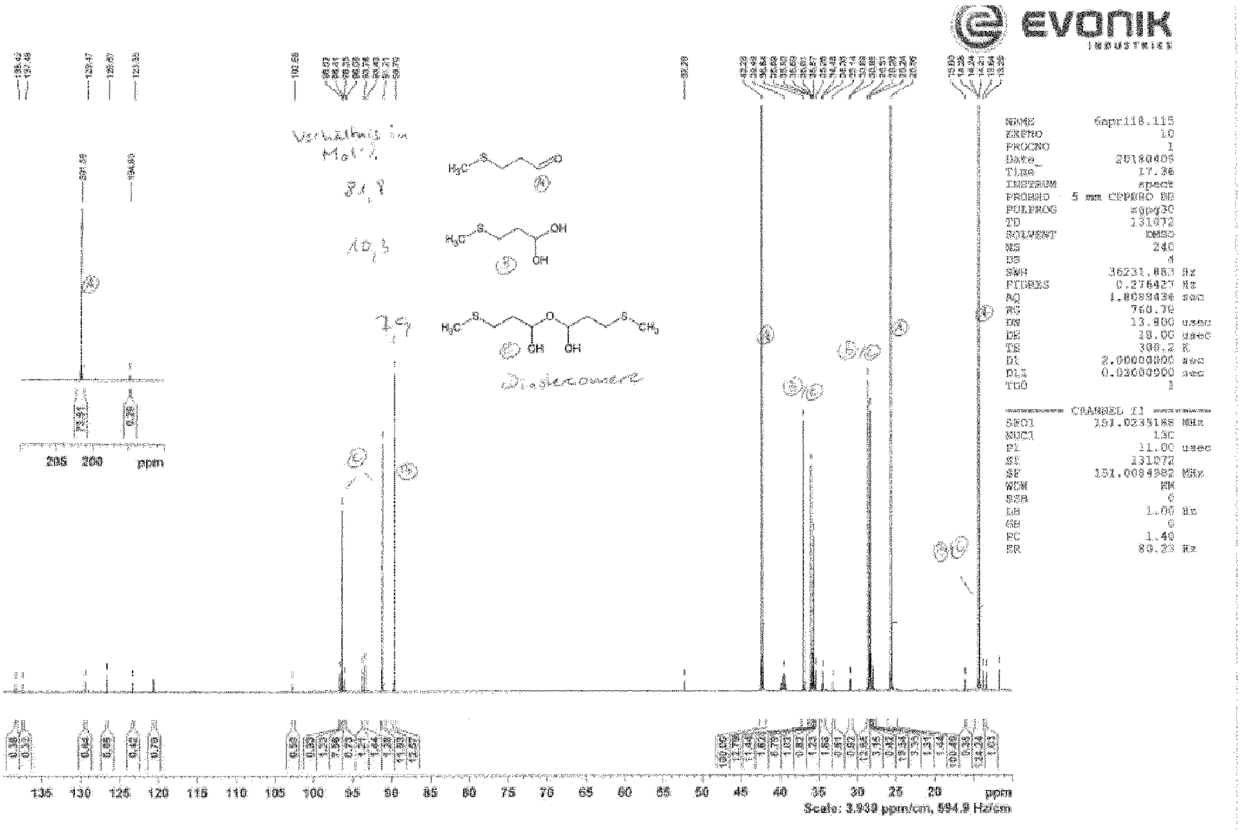

Figur 3: Beispielhaftes 1H NMR-Spektrum einer erfindungsgemäßen Zusammensetzung

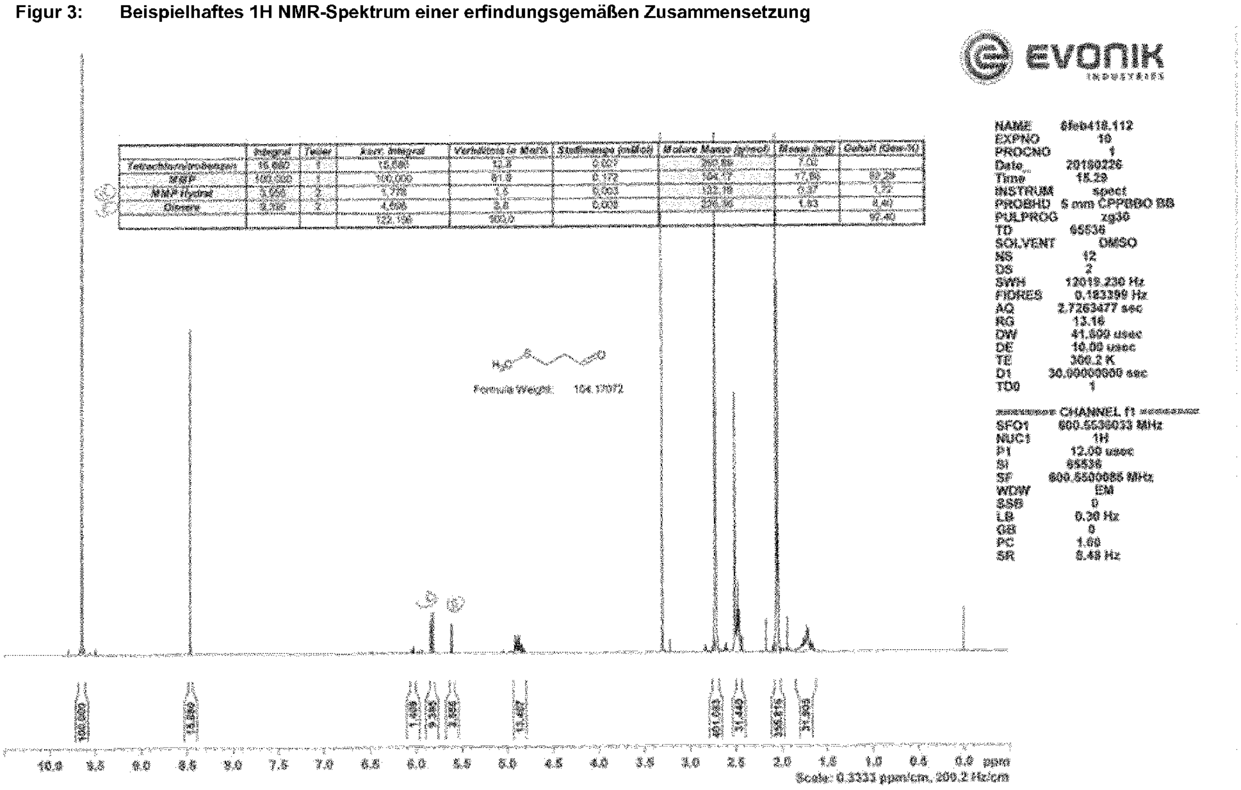

Europäisches Patentamt
European Patent Office
Office européen des brevets

# EUROPÄISCHER RECHERCHENBERICHT

Nummer der Anmeldung
EP 18 20 7569

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | Christian Fernández-García ET AL: "S1 Supporting Information for Prebiotic Synthesis of Aminooxazoline-5'-Phosphates in Water by Oxidative Phosphorylation", , 1. Januar 2017 (2017-01-01), XP055583737, Gefunden im Internet: URL:http://www.rsc.org/suppdata/c7/cc/c7cc02183f/c7cc02183f1.pdf [gefunden am 2019-04-26] * Seite S35 * | 1-15 | INV. C07C319/12 C07C323/12 |
| Y,D | EP 3 339 290 A1 (EVONIK DEGUSSA GMBH [DE]) 27. Juni 2018 (2018-06-27) * das ganze Dokument * | 1-15 | |
| Y | US 4 048 232 A (KOBERSTEIN EDGAR ET AL) 13. September 1977 (1977-09-13) * Spalte 2 * | 1-15 | |
| Y | CN 102 399 177 B (KUANYI LI) 24. Februar 2016 (2016-02-24) * Zusammenfassung * | 1-15 | RECHERCHIERTE SACHGEBIETE (IPC) C07C |

Der vorliegende Recherchenbericht wurde für alle Patentansprüche erstellt

1

| Recherchenort | Abschlußdatum der Recherche | Prüfer |
|---|---|---|
| Den Haag | 2. Mai 2019 | Tabanella, Stefania |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 18 20 7569

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-05-2019

| Im Recherchenbericht angeführtes Patentdokument | Datum der Veröffentlichung | Mitglied(er) der Patentfamilie | Datum der Veröffentlichung |
|---|---|---|---|
| EP 3339290 A1 | 27-06-2018 | BR 102017027766 A2 | 04-12-2018 |
| | | CN 108218749 A | 29-06-2018 |
| | | EP 3339290 A1 | 27-06-2018 |
| | | JP 2018104415 A | 05-07-2018 |
| | | SG 10201710613W A | 30-07-2018 |
| | | US 2018179155 A1 | 28-06-2018 |
| US 4048232 A | 13-09-1977 | AT 331773 B | 25-08-1976 |
| | | BE 813990 A | 21-10-1974 |
| | | CA 1005460 A | 15-02-1977 |
| | | CH 582665 A5 | 15-12-1976 |
| | | DD 110862 A5 | 12-01-1975 |
| | | DE 2320544 B1 | 12-09-1974 |
| | | ES 423736 A1 | 16-04-1976 |
| | | FR 2226393 A1 | 15-11-1974 |
| | | GB 1400702 A | 23-07-1975 |
| | | IT 1005995 B | 30-09-1976 |
| | | JP S5012012 A | 07-02-1975 |
| | | NL 7404691 A | 23-10-1974 |
| | | RO 68025 A | 30-12-1980 |
| | | SE 397344 B | 31-10-1977 |
| | | SU 505357 A3 | 28-02-1976 |
| | | US 4048232 A | 13-09-1977 |
| CN 102399177 B | 24-02-2016 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- GB 1618884 A
- GB 1173174 A
- GB 1166961 A
- WO 9313059 A
- EP 899258 A1
- JP 49116017 A
- JP 10152467 A
- EP 2813489 A1
- EP 3205643 A1
- EP 3339290 A1
- EP 143100 B1
- EP 2678313 A1
- EP 2768312 A1
- US 5705675 A
- EP 780370 A2

### In der Beschreibung aufgeführte Nicht-Patentliteratur

- **C. FERNANDEZ-GARCIA.** *Chem. Commun.,* 2017, vol. 53, 4919
- **P. A. BRUTTEL ; R. SCHLINK.** Wasserbestimmung durch Karl-Fischer-Titration. Metrohm AG, 2006